Europäisches Patentamt

European Patent Office

Office européen des brevets

(11) Veröffentlichungsnummer: **0 163 000 B1**

(12) **EUROPÄISCHE PATENTSCHRIFT**

(45) Veröffentlichungstag der Patentschrift: **03.04.91**

(51) Int. Cl.⁵: **A61K 9/52**, A61K 9/54

(21) Anmeldenummer: **85100375.6**

(22) Anmeldetag: **16.01.85**

(54) **Pharmazeutisches Produkt in Form von Pellets mit kontinuierlicher, verzögerter Wirkstoffabgabe.**

(30) Priorität: **01.02.84 DE 3403329**

(43) Veröffentlichungstag der Anmeldung:
**04.12.85 Patentblatt 85/49**

(45) Bekanntmachung des Hinweises auf die
Patenterteilung:
**03.04.91 Patentblatt 91/14**

(84) Benannte Vertragsstaaten:
**AT BE CH DE FR GB IT LI LU NL SE**

(56) Entgegenhaltungen:
**WO-A-82/01468**
**DE-A- 1 949 894**
**GB-A- 800 973**
**US-A- 2 928 770**

(73) Patentinhaber: **3 M Medica GmbH**

**W-4280 Borken/Westf.(DE)**

(72) Erfinder: **Zerbe, Horst, Dr.**
**Wilhelm-Busch-Strasse 4**
**W-4282 Velen(DE)**

(74) Vertreter: **Cohausz & Florack Patentanwälte**
**Postfach 14 01 61 Schumannstrasse 97**
**W-4000 Düsseldorf 1(DE)**

**Beschreibung**

Die vorliegende Erfindung betrifft ein pharmazeutisches Produkt in Pelletform mit kontinuierlicher, aber verzögerter Wirkstoffabgabe.

Pharmazeutische Wirkstoffe werden in beträchtlichem Umfang aus sogenannten Pellets verabreicht. Zur Erreichung einer verzögerten Wirkstoffabgabe wird das wirkstoffhaltige Pellet mit einer Lackschicht aus einen solchen Material überzogen, daß der Magen-Darm-Saft den Wirkstoff aus dem Pellet nur langsam nach den Diffusionsgesetzen helauslöst. In bedeutenden Umfang besteht dabei das Pellet aus vorgefertigten Kernen aus im Magen-Darm-Saft löslichem Material z.B. mit Stärke oder dergleichen verbundenen Zuckerkristallen (Saccharose-Kristallen), auf die dann die wirkstoffhaltige Depotschicht und schließlich die die möglichst kontinuierliche, verzögerte Wirkstoffabgabe bewirkende Lackschicht aufgetragen wird. Schließlich ist dann noch häufig eine äußere wirkstoffhaltige Schicht aufgetragen, aus der der Wirkstoff nach Einnahme des Pellet unverzüglich abgegeben wird und somit eine Initialdosis des Wirkstoffes zur Erreichung eines bestimmten gewünschten Wirkstoff-Blutspiegels freigibt. Aufgabe dieser Zubereitungsform ist es, gegebenenfalls nach Erreichung eines solchen gewünschten Ausgangswirkstoffblutspiegels beim Patienten, für einen vorgegebenen Zeitraum, z.B. von mehreren Stunden, möglichst kontinuierlich weiteren Wirkstoff verzögert, unter Aufrechterhaltung des gewünschten Blutspiegels im wesentlichen über den gesamten Zeitraum, und im wesentlichen vollständig aus dem Produkt abzugeben. Die Lösung dieser Aufgabe wird bei derartigen Pellets jedoch dadurch gestört, daß die die möglichst kontinuierliche verzögerte Wirkstoffabgabe bewirkende Lackschicht nach Einnahme der Pellets vorzeitig reißt, so daß der noch im Pellet enthaltene Wirkstoff durch die Bruchstellen entgegen der angestrebten Kontinuität und derart verzögerten Wirkstoffabgabe, daß Wirkstoff über den ganzen vorgegebenen Zeitraum unter Erhalt des gewünschten Blutspiegels abgegeben wird, vorzeitig und diskontinuierlich freigegeben wird. Man hat zahlreiche Versuche in der pharmazeutischen Industrie unternommen, dieses Problem zu lösen. So wurde das Material der äußeren Lackschicht variiert. Eine Beseitigung des Problems bei derartigen Pellets wurde jedoch hierdurch nicht erreicht.

Das erfindungsgemäße pharmazeutische wirkstoffhaltige Produkt in Form von Pellets mit für einen vorgegebenen Zeitraum möglichst kontinuierlicher, verzögerter, im wesentlichen vollständiger Abgabe des enthaltenen Wirkstoffs besteht wie bisher aus einem Kern aus im Magen-Darm-Saft löslichem Material, einer diesen umschließenden wirkstoffhaltigen Depotschicht und einer die für den vorgesehenen Zeitraum kontinuierliche, verzögerte Wirkstoffabgabe kontrollierenden und bewirkenden Lackschicht und gegebenenfalls einer weiteren wirkstoffhaltigen, gegen den Magen-Darm-Saft ungeschützten äußeren Initialschicht zur Erreichung eines gewünschten Ausgangswirkstoffblutspiegels und ist dadurch gekennzeichnet, daß zwischen der wirkstoffhaltigen Depotschicht und dem Kern aus im Magen-Darm-Saft löslichem Material eine diesen Kern für den Magen-Darm-Saft undurch lässig und dicht umschließende Isolierschicht aus im Magen-Darm-Saft unlöslichem Material vorgesehen ist.

Im Magen-Darm-Saft unlösliche Materialien sind dem Fachmann bekannt. Ganz besonders bevorzugt besteht die Zwischenschicht aus im Magen-Darm-Saft unlöslichem Material aus Ethylcellulose mit einem Ethoxylgehalt von 47,5 bis 49 %. Weitere besonders geeignete Materialien sind Polyvinylacetat, anionische Polymerisate aus Methacrylsäure und Methacrylsäureestern, Acryl- und Methacrylsäureester-Copolymerisate, Schellack sowie Mischungen dieser Substanzen. Der Kern der erfindungsgemäßen Pellets besteht wie bisher aus Saccharose oder durch Stärkesirup verbundene Saccharose.

Die die wirkstoffhaltige Depotschicht umschließende und die für den vorgesehenen Zeitraum kontinuierliche, verzögerte Wirkstoffabgabe kontrollierende und bewirkende Lackschicht besteht bevorzugt aus den gleichen Grundstoffen wie die zuvor beschriebene Isolierschict aus im Magen-Darm-Saft unlöslichem Material. Sie enthält zusätzlich porenbildende Stoffe, die dem Fachmann ebenfalls bekannt sind. Ganz besonders bevorzugt wird hierfür Polyethylenglykol verwendet. Weitere geeignete Materialien sind Polyvinylpyrrolidon, Hydroxypropylmethylcellulose, Hydroxypropylcellulose sowie Mischungen dieser Stoffe. Art und Menge dieser Zusätze hängen in bekannter Weise von der Geschwindigkeit ab, mit der der Wirkstoff aus der Depotschicht freigesetzt werden soll.

Wirkstoffe, für die das erfindungsgemäße Produkt insbesondere Anwendung finden kann, sind, ohne die Erfindung zu begrenzen, Isosorbid-5-nitrat, Isosorbiddinitrat und Captopril.

Um den Verbund der Depotschicht und der Initialschicht zu gewährleisten und die Zerstörung dieser Schichten durch mechanische Belastung zu verhindern, können während der Erzeugung dieser Schichten wasserlösliche klebende Polymere verwendet werden. Die hierfür in Frage kommenden physiologisch unbedenklichen Substanzen sind dem Fachmann bekannt.

Überraschenderweise zeigen die erfindungsgemäßen Pellets, nach einer sofortigen Initialdosis bzw. Dosis zur Erreichung eines gewünschten Ausgangswirkstoffblutspiegels im Patienten, sofern eine ungeschützte wirkstoffhaltige äußere Initialschicht hier vorgesehen ist, eine praktisch vollständig kontinuierliche, verzögerte Wirkstoffabgabe und dazu eine im wesentlichen vollständige Abgabe des in dem Pellet enthaltenen Wirkstoffes, d.h. mit dem erfindungsgemäßen Pellet wird zuerst der gewünschte Ausgangswirkstoffblutspiegel erreicht und das Pellet gibt dann kontinuierlich im wesentlichen soviel Wirkstoff ab, daß dieser gewünschte Wirkstoffblutspiegel aufrechterhalten bleibt.

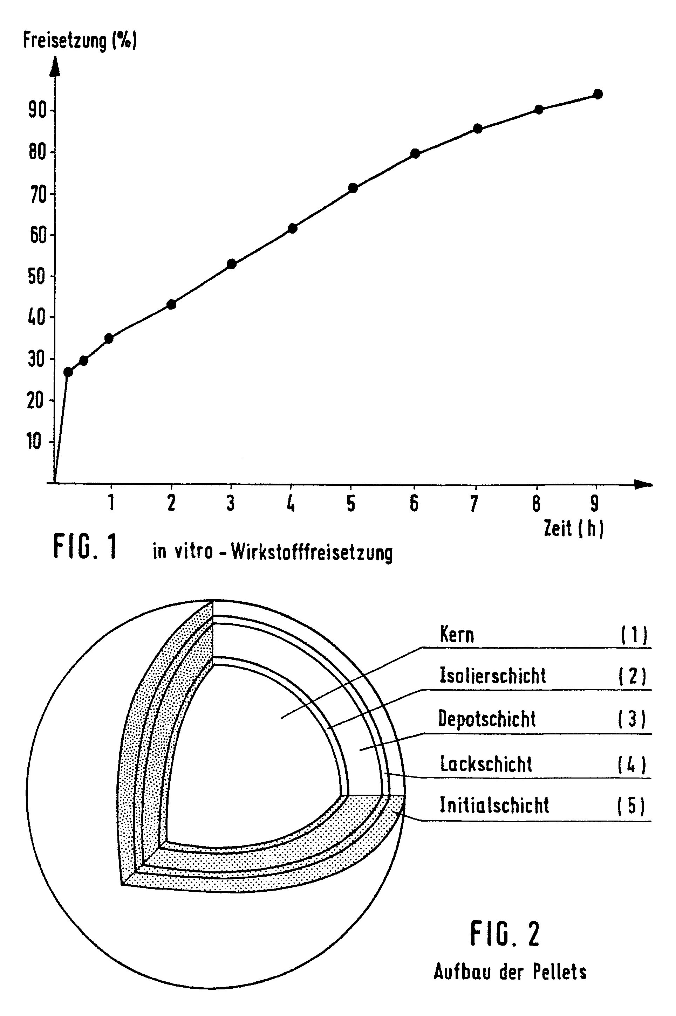

Dies geht aus dem Diagramm gemäß Figur 1 für einen erfindungsgemäßen Pellet für Isosorbid-5-nitrat (5-ISN) hervor. Wie hieraus ersichtlich, ist eine praktisch vollständig kontinuierliche Wirkstoffzufuhr im Patienten möglich, ohne daß er laufend über die vorgesehene Zeit mehrmals andere Darreichungsformen zu sich nimmt.

Figur 2 zeigt den Aufbau des erfindungsgemäßen Pellet. Der Kern 1 aus Zuckerkristallen ist von der Isolierschicht 2 aus Ethylcellulose mit einem Ethoxylgehalt von 47,5 bis 49 % dicht umschlossen, so daß kein Magen-Darm-Saft in den Zuckerkern 1 eindringen kann. Der Wirkstoff ist in der Depotschicht 3 enthalten, die wiederum von der Lackschicht 4 umgeben ist, die zusätzlich zu dem Lackmaterial der Isolierschicht 2 Polyethylenglykol in einer Menge von ca. 10 % als Porenbildner enthält. Die Depotschicht 3 enthält 70 % der insgesamt im Pellet enthaltenden Menge an dem Wirkstoff Isosorbid-5-nitrat. Die äußere Initialschicht 5, die gegenüber der Einwirkung von Magen-Darm-Saft ungeschützt ist, enthält 30 % des Wirkstoffes, damit nach Einnahme des Pellets im Blutspiegel des Patienten die gewünschte Höhe erreicht wird.

Beispiel 1:

Ein pharmazeutisches Produkt gemäß der vorliegenden Erfindung wird wie folgt hergestellt:

Eine Suspension bestehend aus 100 g Ethylcellulose, 20 g Talkum, 475 ml Ethylalkohol, 475 ml Methylenchlorid wird mit Hilfe einer Sprüheinrichtung auf 1000 g Zuckerkügelchen mit einem Teilchendurchmesser von 0,6 - 0,71 mm aufgesprüht.

Anschließend werden 1500 g einer Mischung aus 9 Teilen Isosorbid-5-nitrat und einem Teil Lactose unter Verwendung einer Klebstofflösung aus 57 g Hydroxypropylcellulose und 570 ml Wasser auf die isolierten Zuckerkügelchen aufgebracht.

Auf 2000 g dieser Pellets wird mit Hilfe einer geeigneten Sprüheinrichtung eine Suspension bestehend aus 196,6 g Ethylcellulose, 19,66 g Polyethylenglykol, 49,2 g Talkum, 950 ml Ethylalkohol, 950 ml Methylenchlorid aufgesprüht. Auf 2000 g dieser Pellets werden 420 g einer Mischung aus 9 Teilen Isosorbid-5-nitrat und einem Teil Lactose mit Hilfe einer Lösung bestehend aus 20 g Hydroxypropylcellulose und 200 ml Wasser aufgebracht.

Wirkstofffreisetzung

An einer Menge der nach Beispiel 1 hergestellten Pellets, die einem Wirkstoffgehalt von ca. 100 mg entspricht, wurde die Wirkstofffreisetzung ermittelt. Um den physiologischen Gegebenheiten Rechnung zu tragen, wurde als Freigabemedium während der ersten 2 Stunden künstlicher Magensaft, anschließend künstlicher Darmsaft verwendet. Das Volumen des Freigabemediums wurde so gewählt, daß über die Dauer des Versuchs sink-Bedingungen eingehalten werden. Nach festgelegten Zeitabständen wurde die freigesetzte Menge an Isosorbid-5-nitrat flüssigkeitschromatographisch bestimmt. Das Ergebnis ist in Figur 1 graphisch dar-gestellt.

**Ansprüche**

1. Pharmazeutisches wirkstoffhaltiges Produkt in Form von Pellets mit für einen vorgesehenen Zeitraum möglichst kontinuierlicher, verzögerter, im wesentlichen vollständiger Abgabe des im Pellet enthaltenen Wirkstoffs, bestehend aus einem Kern (1) aus im Magen-Darm-Saft löslichem Material, einer diesen umschließenden, wirkstoffhaltigen Depotschicht (3) und einer die für den vorgesehenen Zeitraum konti-nuierlich, verzögerte Wirkstoffabgabe kontrol-lierenden und bewirkenden Lackschicht (4) so-wie gegebenenfalls einer wirkstoffhaltigen, ge-gen den Einfluß des Magen-Darm-Saftes ungeschützten äußeren Initialschicht (5) zur Erreichung einer Initialdosis bzw. eines Ausgangswirkstoffblutspiegels im Patienten, **dadurch gekennzeichnet**, daß zwischen der wirkstoffhaltigen Depotschicht (3) und dem Kern (1) aus im Magen-Darm-Saft löslichem Material eine diesen Kern für Magen-Darm-Saft un-durchlässig und dicht umschließende Isolier-schicht (2) aus einem im Magen-Darm-Saft un-löslichem Material vorgesehen ist.

2. Pharmazeutisches Produkt in Form von Pellets gemäß Anspruch 1, dadurch gekennzeichnet, daß die Isolierschicht (2) aus Ethylcellulose mit einem Ethoxylgehalt t von 47,5 bis 49 %, Polyvinylacetat, einem anionischen Polymerisat aus Methacrylsäure und Methacrylsäureestern, ei-nem Acryl- und Methacrylsäureester-Copoly-merisat, Schellack sowie Mischungen dieser Substanzen besteht.

3. Pharmazeutisches Produkt in Form von Pellets gemäß Anspruch 2, dadurch gekennzeichnet, daß die Isolierschicht (20 aus Ethylcellulose mit einem Ethoxylgehalt von 47,5 bis 49 % besteht.

4. Pharmazeutisches Produkt in Form von Pellets gemäß Anspruch 1 bis 3, dadurch gekennzeichnet, daß die Isolierschicht (2) aus dem gleichen Material wie die kontinuierliche, verzögerte Wirkstoffabgabe kontrollierende und bewirkende Lackschicht (4) mit dem Unterschied besteht, daß in letzterer ein porenbildender Stoff zur kontrollierten verzögerten Wirkstoffabgabe in gewünschtem Umfang in an sich bekannter Menge eingemischt ist.

5. Pharmazeutisches Produkt in Form von Pellets gemäß Anspruch 4, dadurch gekennzeichnet, daß als porenbildender Stoff Polyethylenglykol verwendet ist.

Patentansprüche für folgende Vertragsstaaten: AT

1. Verfahren zur Herstellung von pharmazeutischen wirkstoffhaltigen Produkten in Form von Pellets mit für einen vorgesehenen Zeitraum möglichst kontinuierlicher, verzögerter, im wesentlichen vollständiger Abgabe des im Pellet enthaltenden Wirkstoffs, indem man auf einen Kern (1) aus im Magen-Darf-Saft löslichem Material eine diesen umschließende, wirkstoffhaltige Depotschicht (3) aufbringt und sodann eine die für den vorgesehenen Zeitraum kontinuierliche, verzögerte Wirkstoffabgabe kontrollierende und bewirkende Lackschicht (4) sowie gegebenenfalls eine wirkstoffhaltige, gegen den Einfluß des Magen-Darm-Saftes ungeschützte äußere Initialschicht (5) zur Erreichung einer Initialdosis bzw. eines Ausgangswirkstoffblutspiegels im Patienten aufträgt, dadurch gekennzeichnet, daß zwischen der wirkstoffhaltigen Depotschicht (3) und dem Kern (1) aus im Magen-Darm-Saft löslichem Material eine diesen Kern für Magen-Darm-Saft undurchlässig und dicht umschließende Isolierschicht (2) aus einem im Magen-Darm-Saft unlöslichen Material vorgesehen wird.

2. Verfahren gemäß Anspruch 1, dadurch gekennzeichnet, daß die Isolierschicht (2) aus Ethylcellulose mit einem Ethoxylgehalt von 47,5 bis 49%, Polyvinylacetat, einem anionischen Polymerisat aus Methacrylsäure und Methacrylsäureestern, einem Acryl- und Methacrylsäureester-Copolymerisat, Schellack sowie Mischungen dieser Substanzen hergestellt wird.

3. Verfahren gemäß Anspruch 2, dadurch gekennzeichnet, daß die Isolierschicht (2) aus Ethylcellulose mit einem Ethoxylgehalt von 47,5 bis 49% hergestellt wird.

4. Verfahren gemäß Anspruch 1 bis 3, dadurch gekennzeichnet, daß die Isolierschicht (2) aus dem gleichen Material wie die kontinuierliche, verzögerte Wirkstoffabgabe kontrollierende und bewirkende Lackschicht (4) mit dem Unterschied hergestellt wird, daß in letzterer ein porenbildender Stoff zur kontrollierten verzögerten Wirkstoffabgabe in gewünschtem Umfang in an sich bekannter Weise eingemischt wird.

5. Verfahren gemäß Anspuch 4, dadurch gekennzeichnet, daß als porenbildender Stoff Polyethylenglykol verwendet wird.

**Claims**

1. Pharmaceutical product containing an active ingredient, in the form of pellets with substantially complete release, prolonged and as continuous as possible over a predetermined period of time, of the active ingredient contained in the pellet, consisting of a core (1) of material soluble in the gastrointestinal juices, a deposit layer (3) containing active ingredient and surrounding said core, and a glaze layer (4) which effects and controls the prolonged release of active ingredient continuously over the intended time period, and also possibly an external initial layer (5) containing active ingredient for achieving an initial dose, or initial level of active ingredient in the patient's blood, said initial layer being unprotected against the influence of the gastrointestinal juices, characterised in that between the deposit layer (3) containing active ingredient, and the core (1) made of material soluble in the gastrointestinal juices, there is provided an isolating layer (2) which is made of material insoluble in the gastrointestinal juices and which surrounds this core in sealing-tight manner and such as to be impervious to gastrointestinal juices.

2. Pharmaceutical product in the form of pellets according to claim 1, characterised in that the isolating layer (2) consists of ethyl cellulose with an ethoxyl content of 47.5 to 49 %, polyvinyl acetate, an anionic polymer of methacrylic acid and methacrylic acid esters, a copolymer of acrylic and methacrylic acid esters, shellac, and mixtures of these substances.

3. Pharmaceutical product in the form of pellets according to claim 2, characterised in that the isolating layer (2) consists of ethyl cellulose with an ethoxyl content of 47.5 to 49 %.

4. Pharmaceutical product in the form of pellets according to claims 1 to 3, characterised in that the isolating layer (2) consists of the same material as the glaze layer (4) which effects and controls the continuous prolonged release of active ingredient, with the difference that a pore-forming substance for controlled prolonged release of active ingredient to the desired extent is mixed in a per se known quantity into the said glaze layer.

5. Pharmaceutical product in the form of pellets according to claim 4, characterised in that polyethylene glycol is used as pore-forming substance.

Claims for the following Contracting States: AT

1. Process for the production of pharmaceutical products containing an active ingredient, in the form of pellets with substantially complete release, prolonged and as continuous as possible, of the active ingredient contained in the pellet, wherein there is applied on to a core (1) made of material soluble in the gastrointestinal juices a deposit layer (3) which contains active ingredient and surrounds said core, and then there are applied a glaze layer (4) effecting and controlling the prolonged release of active ingredient continuously over the predetermined period of time, and also possibly an external initial layer (5) for achieving an initial dose or an initial level of active ingredient in the patient's blood, said initial layer containing active ingredient and being unprotected against the influence of the gastrointestinal juices, characterised in that between the deposit layer (3) containing active ingredient, and the core (1) made of material soluble in the gastrointestinal juices there is provided an insulating layer (2) which is made of material insoluble in the gastrointestinal juices and which surrounds this core in sealing-tight manner and such as to be impervious to gastrointestinal juices.

2. Process according to claim 1, characterised in that the isolating layer (2) is made from ethyl cellulose with an ethoxyl content of 47.5 to 49 %, polyvinyl acetate, an anionic polymer of methacrylic acid and methacrylic acid esters, a copolymer of acrylic and methacrylic acid esters, shellac, and mixtures of these substances.

3. Process according to claim 2, characterised in that the isolating layer (2) is made from ethyl cellulose with an ethoxyl content of 47.5 to 49 %

4. Process according to claims 1 to 3, characterised in that the isolating layer (2) is produced from the same material as the glaze layer (4) which effects and controls the continuous prolonged release of active ingredient, with the difference that a pore-forming substance for controlled prolonged release of active ingredient to the desired extent is mixed in a per se known manner into the said glaze layer.

5. Process according to claim 4, characterised in that polyethylene glycol is used as pore-forming substance.

**Revendications**

1. Produit pharmaceutique contenant une substance active se présentant sous forme de capsules permettant une libération prolongée, aussi continue que possible pendant un temps prédéterminé essentiellement totale de la substance active contenue dans la capsule, constitué d'un noyau (1) réalisé en un matériau soluble dans le suc gastro-intestinal, d'une couche de dépôt (3) contenant la substance active l'entourant et d'une couche de laque (4) provoquant et contrôlant la libération continue et prolongée de la substance active pendant le temps prévu ainsi qu'éventuellement d'une couche initiale extérieure (5) contenant de la substance active, non protégée contre l'action du suc gastro-intestinal, pour parvenir à une dose initiale ou au taux de départ désiré de substance active dans le sang chez le patient, caractérisé en ce qu'il est prévu, entre la couche de dépôt (3) contenant la substance active et le noyau (1) réalisé en un matériau soluble dans le suc gastro-intestinal, une couche isolante (2) réalisée en un matériau insoluble dans le suc gastro-intestinal, entourant ledit noyau, de façon serrée et imperméable au suc gastro-intestinal.

2. Produit pharmaceutique se présentant sous forme de capsules selon la revendication 1, caractérisé en ce que la couche isolante (2) est constituée d'éthyl-cellulose ayant une teneur en éthoxy comprise entre 47,5 et 49%, d'acétate de polyvinyle, d'un polymérisat anionique d'acide méthacrylique et d'esters de l'acide méthacrylique, d'un copolymérisat d'esters d'acides acrylique et méthacrylique, de gomme laque ainsi que des mélanges de ces substances.

3. Produit pharmaceutique se présentant sous forme de capsules selon la revendication 1, caractérisé en ce que la couche isolante (2) est constituée d'éthyl-cellulose ayant une teneur en éthoxy comprise entre 47,5 et 49%.

4. Produit pharmaceutique se présentant sous forme de capsules selon les revendications 1 à 3, caractérisé en ce que la couche isolante (2) est constituée du même matériau que la couche de laque (4) provoquant et contrôlant la libération continue et prolongée de la substance active, avec cette différence que l'on introduit dans cette dernière couche un agent porogène, dans la mesure souhaitée, en une quantité connue en soi, pour la libération contrôlée et prolongée de la substance active.

5. Produit pharmaceutique se présentant sous forme de capsules selon la revendication 4, caractérisé en ce que l'on utilise comme agent porogène le polyéthylèneglycol.

Revendications pour les Etats contractants suivants: AT

1. Procédé de préparation d'un produit pharmaceutique contenant une substance active se présentant sous forme de capsules permettant une libération prolongée, aussi continue que possible pendant un temps prédéterminé, essentiellement totale de la substance active contenue dans la capsule, selon lequel on dépose sur un noyau (1) réalisé en un matériau soluble dans le suc gastro-intestinal, une couche de dépôt (3) contenant la substance active l'entourant et ensuite une couche de laque (4) provoquant et contrôlant la libération continue et prolongée de la substance active pendant le temps prévu ainsi qu'éventuellement une couche initiale extérieure (5) contenant de la substance active, non protégée contre l'action du suc gastro-intestinal, pour parvenir à une dose initiale ou au taux de départ désiré de substance active dans le sang chez le patient, caractérisé en ce qu'il est prévu, entre la couche de dépôt (3) contenant la substance active et le noyau (1) réalisé en un matériau soluble dans le suc gastro-intestinal, une couche isolante (2) réalisée en un matériau insoluble dans le suc gastro-intestinal, entourant ledit noyau, de façon serrée et imperméable au suc gastro-intestinal.

2. Procédé selon la revendication 1, caractérisé en ce que la couche isolante (2) est fabriquée à partir d'éthyl-cellulose ayant une teneur en éthoxy comprise entre 47,5 et 49 %, d'acétate de polyvinyle, d'un polymérisat anionique d'acide méthacrylique et d'esters de l'acide méthacrylique, d'un copolymérisat d'esters d'acides acrylique et méthacrylique, de gomme laque ainsi que des mélanges de ces substances.

3. Procédé selon la revendication 2, caractérisé en ce que la couche isolante (2) est fabriquée à partir d'éthylcellulose ayant une teneur en éthoxy comprise entre 47,5 et 49 %.

4. Procédé selon les revendications 1 à 3, caractérisé en ce que la couche isolante (2) est fabriquée à partir du même matériau que la couche de laque (4) provoquant et contrôlant la libération continue et prolongée de la substance active, avec cette différence que l'on introduit dans cette dernière couche un agent porogène, dans la mesure souhaitée, en une quantité connue en soi, pour la libération contrôlée et prolongée de la substance active.

5. Procédé selon la revendication 4, caractérisé en ce que l'on utilise comme agent porogène le polyéthylèneglycol.

Freisetzung (%)

**FIG. 1**   in vitro - Wirkstofffreisetzung

Kern            (1)

Isolierschicht   (2)

Depotschicht     (3)

Lackschicht      (4)

Initialschicht   (5)

**FIG. 2**

Aufbau der Pellets